# FASCICULE DE BREVET EUROPEEN

(11) **EP 0 734 370 B1**
(45) Date de publication et mention de la délivrance du brevet: **20.10.1999**
(21) Numéro de dépôt: 95932137.3
(22) Date de dépôt: 06.10.1995
(51) Int. Cl.: C07C 29/14, C07C 29/143, C07C 29/147

(54) **PROCEDE POUR LA PREPARATION D'ALCOOLS**
VERFAHREN ZUR HERSTELLUNG VON ALKOHOLEN
METHOD FOR PREPARING ALCOHOLS

(30) Priorité: 19.10.1994 CH 313894; 16.02.1995 CH 44595
(43) Date de publication de la demande: 02.10.1996
(73) Titulaire: FIRMENICH SA, 1211 Genève 8 (CH)
(72) Inventeur: MIMOUN, Hubert, F-01630 Challex (FR)
(74) Mandataire: Salvaterra-Garcia, Maria de Lurdes
(86) Numéro de dépôt international: IB9500836
(87) Numéro de publication internationale: WO9612694

(56) Documents cités:
- US-A- 3 061 424
- US-A- 5 220 020
- ANGEWANDTE CHEMIE, vol. 69, 1957 WEINHEIM, DE, page 96 S. NITZSCHE, ET AL.: 'Reduktionen mit Methyl-wasserstoff-polysiloxanen' cité dans la demande
- JOURNAL OF ORGANIC CHEMISTRY, vol. 34, no. 6, Juin 1969 WASHINGTON, DC, US, pages 2014-2016, G.L. GRADY, ET AL.: 'A simple technique for performing reactions with organotin hydrides' cité dans la demande
- JOURNAL OF ORGANIC CHEMISTRY, vol. 38, no. 1, 12 Janvier 1973 WASHINGTON, DC, US, pages 162-165, J. LIPOWITZ, ET AL.: 'Use of polymethylhydrosiloxane as a selective, neutral reducing agent for aldehydes, ketones, olefins, and aromatic nitro compounds' cité dans la demande
- JOURNAL OF ORGANIC CHEMISTRY, vol. 59, no. 15, 29 Juillet 1994 WASHINGTON, DC, US, pages 4323-4226, K.J. BARR, ET AL.: 'Titanocene-catalysed reduction of esters using polymethylhydrosiloxane as the stoichiometric reductant' cité dans la demande
- JOURNAL OF THE CHEMICAL SOCIETY, CHEMICAL COMMUNICATIONS, no. 12, 1984 LETCHWORTH, GB, pages 798-799, S.A. MATLIN, ET AL.: 'An immobilised organotin catalyst for reduction of ketones and aldehydes'
- TETRAHEDRON, vol. 37, no. 11, 1981 OXFORD, GB, pages 2165-2171, J. BOYER, ET AL.: 'Réduction selective de composés carbonylés par catalyse hétérogène à la surface des sels'

## Description

La réduction de composés carbonylés tels qu'aldéhydes, cétones, esters ou lactones représente une réaction de choix pour la préparation générale d'alcools. Dans ce contexte, la réduction de la fonction carbonyle dans les composés présentant d'autres groupes insaturés dans la molécule, telles que des fonctions carbone-carbone éthyléniques ou acétyléniques, présente des difficultés car les méthodes d'hydrogénation catalytique par l'hydrogène sont généralement peu sélectives. Il en va de même pour la réduction de composés carbonylés possédant une configuration spatiale définie. En effet l'emploi de catalyseurs ordinaires tels par exemple les chromites de cuivre n'agissent qu'à des températures et des pressions élevées et entraînent une réduction des autres fonctions présentes et, dans bien des cas, une modification de la stéréochimie.

Dans les cas de la réduction des composés carbonylés insaturés, seul l'aluminohydrure de lithium possède la propriété de réduire à la fois aldéhydes, cétones et esters dans des conditions de réaction douces tout en étant inerte vis-à-vis des liaisons insaturées carbone-carbone éventuellement présentes dans la molécule. Le borohydrure de sodium ou le NaAlH₂ (OCH₂CH₂OCH₃)₂, [Vitride®] également employés pour promouvoir la réduction de la fonction carbonyle, sont peu réactifs vis-à-vis des esters. Tous ces réactifs, dont l'emploi nécessite des quantités stoechiométriques, présentent l'inconvénient majeur d'être très sensibles à l'humidité et à l'action de l'air; de plus, ces réactifs sont très coûteux et polluants, d'où la nécessité de recourir à d'autres systèmes plus économiques et d'emploi plus aisé.

Il est connu que le polyméthyl-hydroxysilane (PMHS), dont la formule générale est définie ci-après :

(CH₃)₃-SiO(CH₃HSiO)ₙSi(CH₃)₃

n étant un nombre entier indiquant le nombre d'unités recourantes, peut promouvoir la réduction d'aldéhydes en alcools lorsqu'il est mis en présence de catalyseurs à base d'étain [voir Nizsche et Wick, Angew. Chem. 1957, 69, 96 et brevet US 3,061,424].

Grady et Kuivila (J. Org. Chem. 1969, 34, 2014) et Lipowitz et Bowman (Aldrichim. Acta 1973, 6, 1 et J. Org. Chem. 1975, 38, 162) ont montré que la catalyse à l'étain pour le système réducteur PMHS pouvait s'appliquer à la réduction des aldéhydes et des cétones, mais pas à celle des esters en alcools.

Le brevet US 5,220,020 décrit une méthode de préparation d'alcools par réduction de composés carbonylés à l'aide d'un système constitué par un agent réducteur silanique et un catalyseur métallique de formule

M(L)(L')(L") à M(L)(L')(L")(L''')(L'^{v})(L^{v})

dans laquelle M est un métal appartenant au groupe 3, 4, 5 ou 6, un lanthanide ou un actinide, tandis que (L') à (L^{v}) représentent l'hydrogène, un alkyle, un aryle, un silyle, un halogène, ou un reste -OR, -SR ou -N R(R') dans laquelle R et R' sont l'hydrogène, un alkyle ou un aryle. Un tel système pouvait s'appliquer à la réduction d'esters, lactones, amides et imides.

Parmi les catalyseurs préférentiels, le brevet précité mentionne l'isopropylate et l'éthylate de titane (IV), ainsi que l'isopropylate de trichlorotitane (IV).

Plus récemment Barr, Berk et Buchwald (J. Org. Chem. 1994, 59, 4323) ont montré que le complexe Cp₂TiCl₂ réduit par du butyllithium ou du bromure d'éthylmagnésium pouvait catalyser la réduction d'esters en alcools correspondants avec de bons rendements, mais cette technique requiert des réactifs catalytiques coûteux et difficiles à utiliser à grande échelle dans le cadre d'une préparation industrielle.

### Exposé de l'invention

Nous avons maintenant découvert qu'il était possible de préparer des alcools avec de bons rendements et de façon économique, par réduction de dérivés carbonylés tels les aldéhydes, les cétones, les esters et les lactones au moyen de l'hydrure de polyméthyl-hydroxysiloxane en présence d'un catalyseur pouvant être préparé in-situ ou séparément à partir d'un sel ou complexe métallique et d'un agent réducteur.

Le procédé de l'invention présente l'avantage d'employer des réactifs peu coûteux, dont l'emploi ne nécessite pas de précautions particulières quant à la protection vis-à-vis de l'humidité ou de l'air. Nous avons également constaté que le système réducteur de l'invention ne donnait pas lieu à des prises en masse du milieu réactionnel, contrairement à ce que nous avons pu observer avec les systèmes de l'art antérieur.

La présente invention a donc pour objet un procédé pour la préparation d'alcools par la réduction de la fonction carbonyle dans des substrats appartenant à la classe des aldéhydes, cétones, esters ou lactones, pouvant contenir ou non des fonctions insaturées autres que le carbonyle, caractérisé en ce qu'il comprend :
a. la réaction du substrat carbonylé avec des quantités stoïchiométriques de PMHS en présence d'un système catalytique préparé à partir d'un sel ou complexe métallique et d'un agent réducteur, ledit sel ou complexe métallique étant de formule générale MXₙ dans laquelle M représente un métal de transition choisi parmi le zinc, le cadmium, le manganèse, le cobalt, le fer, le cuivre, le nickel, le ruthénium et le palladium, X un anion de type halogénure, carboxylate ou un ligand anionique, et n un nombre de 1 à 4 ; et l'agent réducteur étant sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens ;
b. l'hydrolyse du siloxane obtenu au moyen d'un agent basique, et
c. la séparation et purification de l'alcool désiré ainsi formé.

La réaction qui caractérise le procédé de l'invention est illustrée par les schémas que voici : 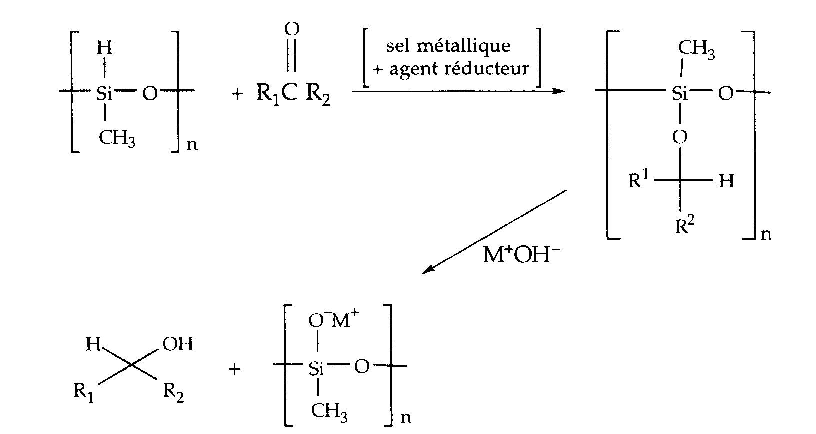 pour les aldéhydes et les cétones, et 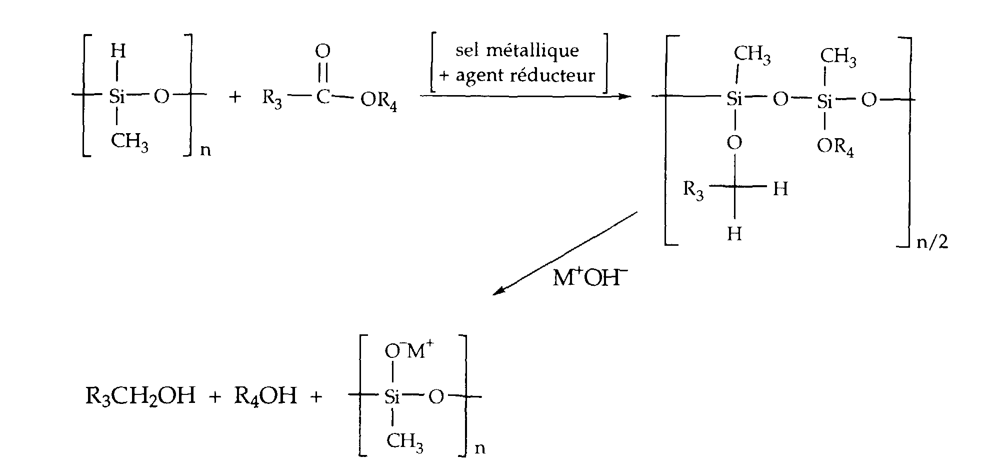 pour les esters et les lactones.

L'invention concerne également les systèmes catalytiques et réducteurs conformes aux revendications et qui sont susceptibles d'être obtenus et/ou utilisés dans ce procédé.

### Mode d'exécution préférentiel de l'invention

Le catalyseur selon l'invention peut être obtenu, in-situ, dans le milieu réactionnel, ou être préparé séparément à partir d'un sel ou complexe métallique de formule générale MXₙ dans laquelle M représente un métal de transition choisi parmi le zinc, le cadmium, le manganèse, le cobalt, le fer, le cuivre, le nickel, le ruthenium et le palladium, X un anion tel qu'un halogénure, un carboxylate ou un ligand anionique quelconque, et n un nombre compris entre 1 et 4. A ce titre on peut utiliser un chlorure, bromure, iodure, carbonate, isocyanate, cyanate, sulfate, phosphate, acétate, propionate, 2-éthylhexanoate, stéarate ou naphténate de l'un des métaux pré-cités que l'on fera réagir, afin de générer le catalyseur actif selon le procédé de l'invention, avec un réducteur tel qu'un hydrure.

A cet effet on utilisera un hydrure alcalin tel l'hydrure de lithium, de sodium ou potassium, ou un hydrure alcalino-terreux tel que l'hydrure de magnésium ou de calcium. On peut également utiliser un hydrure de bore tel que BH₃, un borohydrure métallique M⁺BH₄ (M⁺ = Li, Na, K) ou M(BH₄)₂ (M = Mg, Zn, Ca), un alkylborane RₙBH₍₄₋ₙ₎M (R = alkyle, n = 1 à 3, M = métal alcalin), un alcoxyborane (RO)ₙBH₍₄₋ₙ₎M (R= alkyle, n = 1 à 3, M = métal alcalin), un hydrure d'aluminium AlH₃, AlHₙR₃₋ₙ (R = alkyle), MAlH₄ (M = Li, Na, K), MAlHₙ(OR)₄₋ₙ (M = Li, Na, K), un organomagnésien de formule RMgX (R = alkyle, X = Cl, Br, I), un organolithien RLi (R = alkyle, par exemple C₁ à C₄ ou aryle).

Nous avons observé qu'il pouvait être avantageux, surtout lorsqu'on prépare le catalyseur ex-situ, de rajouter au système catalytique constitué du sel métallique et de l'agent réducteur, un ligand à même de complexer l'hydrure métallique formé et de ce fait de mieux le solubiliser en phase organique. Comme ligand on peut utiliser un éther alcool tel que le méthoxyéthanol, ou une amine alcool telle que la diméthylaminométhanol, la diéthanolamine ou la triéthanolamine.

Selon un mode d'exécution particulier de l'invention, on peut par exemple constituer une solution catalytique stable et homogène en faisant réagir dans un solvant organique inerte, par exemple le toluène, le tétrahydrofuranne ou l'éther isopropylique, un équivalent de 2-éthylhexanoate de zinc avec deux équivalents de Vitride® et un équivalent de diméthylaminoéthanol. La réaction est caractérisée par un dégagement d'hydrogène. Une fois ce dégagement terminé on obtient une solution concentrée homogène qui peut être stockée pour son emploi dans le procédé de l'invention.

La concentration du système [sel métallique + agent réducteur], exprimée en % molaire de métal par rapport au substrat, est généralement comprise entre 0,1 et 10%, de préférence entre 1 et 5%.

Le rapport molaire de l'agent réducteur par rapport au métal est par contre généralement compris entre environ 1 et 2.

Lorsque le système catalytique est préparé in-situ, on fera réagir le dérivé métallique choisi avec l'agent réducteur dans un solvant approprié. Après dégagement complet de l'hydrogène formé on introduira le substrat carbonylé à réduire et on ajoutera l'agent silanique dans la solution.

La consommation typique de PMHS sera de 2 équivalents pour la réduction d'esters ou lactones et d'un équivalent pour la réduction d'aldéhydes ou cétones. L'alcool obtenu comme produit de la réduction pourra être séparé par hydrolyse du siloxane obtenu, hydrolyse qui peut s'effectuer en faisant réagir le milieu réactionnel avec une solution aqueuse ou alcoolique d'un agent basique, tel que par exemple de la soude, de la potasse, de la chaux ou du carbonate de sodium. La proportion de base par rapport au PMHS est comprise entre 1 et 2 équivalents molaires. Lorsque l'hydrolyse est terminée, on observe généralement la constitution de deux phases. L'alcool désiré se trouvant dans la phase organique, peut être obtenu par simple évaporation du solvant qui l'accompagne, le résidu obtenu pouvant être soumis à distillation pour purification ultérieure.

Comme indiqué plus haut, un des avantages du procédé de l'invention est qu'il ne donne lieu à aucune prise en masse et qu'il est possible d'opérer en solution très concentrée en l'absence de solvant. Toutefois, afin de mieux contrôler la température de la réaction (exothermique), on préfère opérer avec un solvant.

A titre de solvant approprié on peut utiliser un éther tel que le méthyltertbutyléther, le diisopropyléther, le dioxanne, le tétrahydrofuranne, l'éthylèneglycoldiméthyléther. On peut également opérer dans un hydrocarbure aliphatique tel que l'heptane, l'éther de pétrole, l'octane, le cyclohexane, ou aromatique tel que le benzène, le toluène, le xylène ou le mésitylène.

Comme indiqué plus haut, le procédé de l'invention permet la réduction de composés carbonylés variés, porteurs ou non d'insaturations autres que le carbonyle, par exemple des fonctions oléfiniques, acétyléniques ou de groupes nitriles lesquels ne sont pas ou peu affectés par la réduction.

Comme exemple de substrats aldéhydiques, on peut citer le butanal, le pentanal, l'hexanal, l'heptanal, l'octanal, le décanal, le dodécanal, sous forme linéaire ou ramifiée. D'autres aldéhydes insaturés susceptibles d'être réduits en alcools insaturés correspondants sont l'acroléine, la méthacroléine, le crotonaldéhyde, le prénal, le citral, le rétinal, l'aldéhyde campholénique, l'aldéhyde cinnamique, l'aldéhyde hexyl-cinnamique, le formyl pinane et le nopal. Les aldéhydes aromatiques tels que le benzaldéhyde, l'aldéhyde cuminique, la vanilline, l'aldéhyde salicylique sont également facilement réduits en alcools correspondants.

Comme exemples de cétones saturées ou insaturées susceptibles d'être réduites par les réducteurs silaniques dans le procédé selon l'invention, on peut citer à titre non limitatif l'hexan-2-one, l'octan-2-one, la nonan-4-one, la dodécan-2-one, la méthyl vinyl cétone, l'oxyde de mésityle, l'acétophénone, la cyclopentanone, la cyclohexanone, la cyclododécanone, la cyclohex-1-én-3-one, l'isophorone, l'oxophorone, la carvone et le camphre.

Comme exemples non limitatifs d'esters et de lactones susceptibles d'être réduits par les agents silaniques selon le procédé de l'invention, on peut citer les acétates, propionates, butyrates, isobutyrates, benzoates d'alkyle ou d'aryle, les acrylates, crotonates d'alkyle ou d'aryle, les cinnamates d'alkyle, le cis-3-hexénoate de méthyle, le sorbate de méthyle, le salicylate de méthyle, le 10-undécylènate de méthyle, l'oléate de méthyle, le linoléate de méthyle, le linolénate de méthyle ou tout mélange d'esters d'acides gras naturels, la caprolactone, la butyrolactone, la dodécalactone, le dicetène et la sclaréolide.

Parmi les esters pouvant être réduits selon le procédé de l'invention figurent également les triglycérides d'acides gras, tels ceux qui constituent les huiles végétales ou animales. Lors de la réduction d'un triglycéride mixte dérivé d'acides gras distincts, l'on peut ainsi obtenir simultanément les alcools saturés ou insaturés correspondants selon le schéma réactionnel que voici:

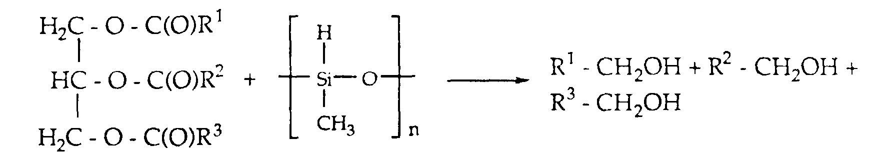

Les substituants R¹, R², et R³ sont généralement des restes hydrocarbonés identiques ou différents pouvant contenir de 1 à 20 atomes de carbone. Lorsque ces restes présentent des insaturations éthyléniques de configuration définie, les alcools résultant conserveront la même stéréochimie. Ainsi, des huiles riches en acide linoléique et linolénique, telles les huiles de lin, se verront transformées en mélange riche en alcools linoléique et linolénique, alors que l'hydrogénolyse conventionnelle des huiles végétales par l'hydrogène gazeux à température et pression élevées en présence de catalyseurs se traduira par une modification de la stéréochimie et de l'emplacement des doubles liaisons des alcools correspondants.

A titre de triglycérides pouvant être réduits par le procédé de l'invention figurent la trioléine, l'huile d'arachide, l'huile de tournesol, l'huile de soja, l'huile d'olive, l'huile de colza, l'huile de sésame, l'huile de lin, l'huile de coton, l'huile de coprah, l'huile de pépins de raisin, l'huile de coco et l'huile de palme par exemple.

La température de réaction est variable et comprise entre 0°C et 150°C, selon la réactivité du substrat. Plus généralement, on opère entre 50°C et 110°C.

L'invention est illustrée par les exemples suivants, dans lesquels les températures sont indiquées en degrés centigrades, les rendements en % molaires, et les abréviations ont le sens usuel de l'art.

### Réduction des aldéhydes

### Exemple 1

### Réduction du trans-hexén-2-al-1

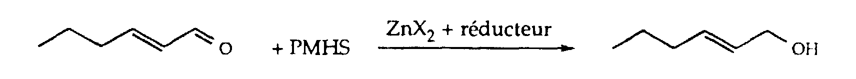

Dans un ballon tricol de 1l, on introduit 50 g d'éther isopropylique, 1,5 g de borohydrure de sodium solide (0,04 mole), puis 11,3 g de 2-éthylhexanoate de zinc (0,04 mole) et on laisse agiter 15 minutes jusqu'à ce que le dégagement d'hydrogène cesse. Puis on introduit 196 g (2 mole) de trans hexén-2-al-1, et on porte le milieu réactionnel à reflux. On introduit alors 147 g de PMHS (2,3 mole) en 2h. On laisse agiter 2 h supplémentaires jusqu'à ce que le contrôle GC indique que tout le substrat a disparu. On refroidit alors le mélange à 20°, on ajoute 100 g d'eau, puis lentement 460 g de soude aqueuse à 30%, en veillant à ce que la température ne dépasse pas 40°.
On décante le mélange, on sépare la phase aqueuse contenant le silicate de sodium, puis on lave la phase organique avec 100 ml d'eau saturée de sel, puis avec 50 g d'une solution aqueuse d'acide acétique à 30%. On évapore le solvant et on obtient 211 g de résidu dont la distillation sur résidus donne 188 g de trans hexén-2-ol-1 de pureté supérieure à 95%.

### Exemples 2 à 13

Ces exemples sont destinés à illustrer l'influence du système catalytique et l'influence du solvant lors de la réduction du trans hexén-2-al-1 en trans hexén-3-ol-1 par le PMHS. On procède comme indiqué à l'exemple 1, mais avec des quantités dix fois inférieures. On charge le solvant, le sel de zinc (2% molaire par rapport au substrat), le réducteur (2% molaire), puis le trans hexén-2-al-1. On procède alors à l'addition en 1 h de 1,1 équivalent de PMHS en maintenant le reflux jusqu'à consommation totale du substrat. On procède alors à l'hydrolyse par de la soude à 30%, puis on récupère l'alcool formé comme dans l'exemple 1. On constate que la plupart des sels de zinc utilisés (ZnCl₂, Zn(2-éthyl-hexanoate)₂, ZnBr₂, ZnEt₂), en combinaison avec un réducteur (LiH, NaH, NaBH₄, LiAlH₄, Vitride®) sont actifs pour la réduction de l'hexén-2-al-1. Dans aucun cas, on ne constate une isomérisation de l'alcool formé, ou la formation de produits secondaires.

| Exemples | Solvant | Sel de zinc 2% molaire | Réducteur 2% molaire | Temps de réaction | Rendement (%) hexén-3-ol-1 |
|---|---|---|---|---|---|
| 2 | méthyl tertbutyl éther | ZnCl₂ | NaBH₄ | 5h | 91% |
| 3 | éther isopropylique | ZnCl₂ | NaBH₄ | 4 h | 94% |
| 4 | éther isopropylique | Zn(2-éthylhexanoate)₂ | NaBH₄ | 5h | 90% |
| 5 | éther isopropylique | Zn(2-éthylhexanoate)₂ | LiH | 6h | 90% |
| 6 | éther isopropylique | Zn(2-éthylhexanoate)₂ | NaH | 6h | 78% |
| 7 | toluène | Zn(2-éthylhexanoate)₂ | NaBH₄ | 4h | 88% |
| 8 | tétrahydro furanne | Zn(2-éthylhexanoate)₂ | NaBH₄ | 5h | 87% |
| 9 | éther isopropylique | ZnBr₂ | NaBH₄ | 6h | 77% |
| 10 | éther isopropylique | Zn(2-éthylhexanoate)₂ | LiAlH₄ | 4 h | 90% |
| 11 | éther isopropylique | Zn(2-éthylhexanoate)₂ | LiAlH₄ | 4h | 89% |
| 12 | éther isopropylique | Zn(2-éthylhexanoate)₂ | Vitride® 1) | 6h | 87% |
| 13 | toluène | ZnEt₂ | LiAlH₄ | 5h | 85% |

| | | | | | |
|---|---|---|---|---|---|
| 1) Vitride ® = NaAlH₂(OCH₂CH₂OMe)₂ | | | | | |

### Exemples 14 à 27

Ces exemples illustrent la possibilité de réduire sélectivement de nombreux aldéhydes en alcools correspondants, sans modification de la stéréochimie de la molécule de départ.
On procède comme indiqué à l'exemple 1, en chargeant dans le réacteur 10 ml d'éther isopropylique, puis 4 mmole de 2-éthyl-hexanoate de zinc puis 4 mmole de NaBH₄, et enfin 0,2 mole de substrat à réduire. On introduit alors en 1 h 0,22 mole de PMHS sous reflux du solvant. Lorsque le substrat a disparu, on procède à l'hydrolyse du milieu réactionnel par de la soude aqueuse à 30%, et on isole l'alcool formé comme dans l'exemple 1.

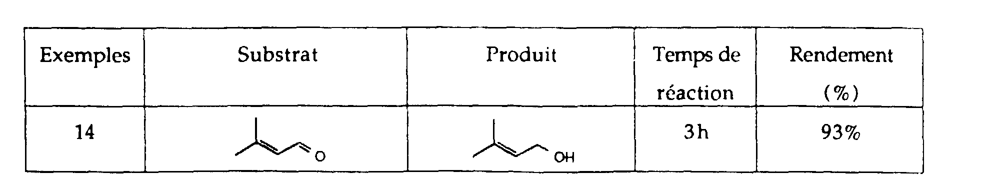

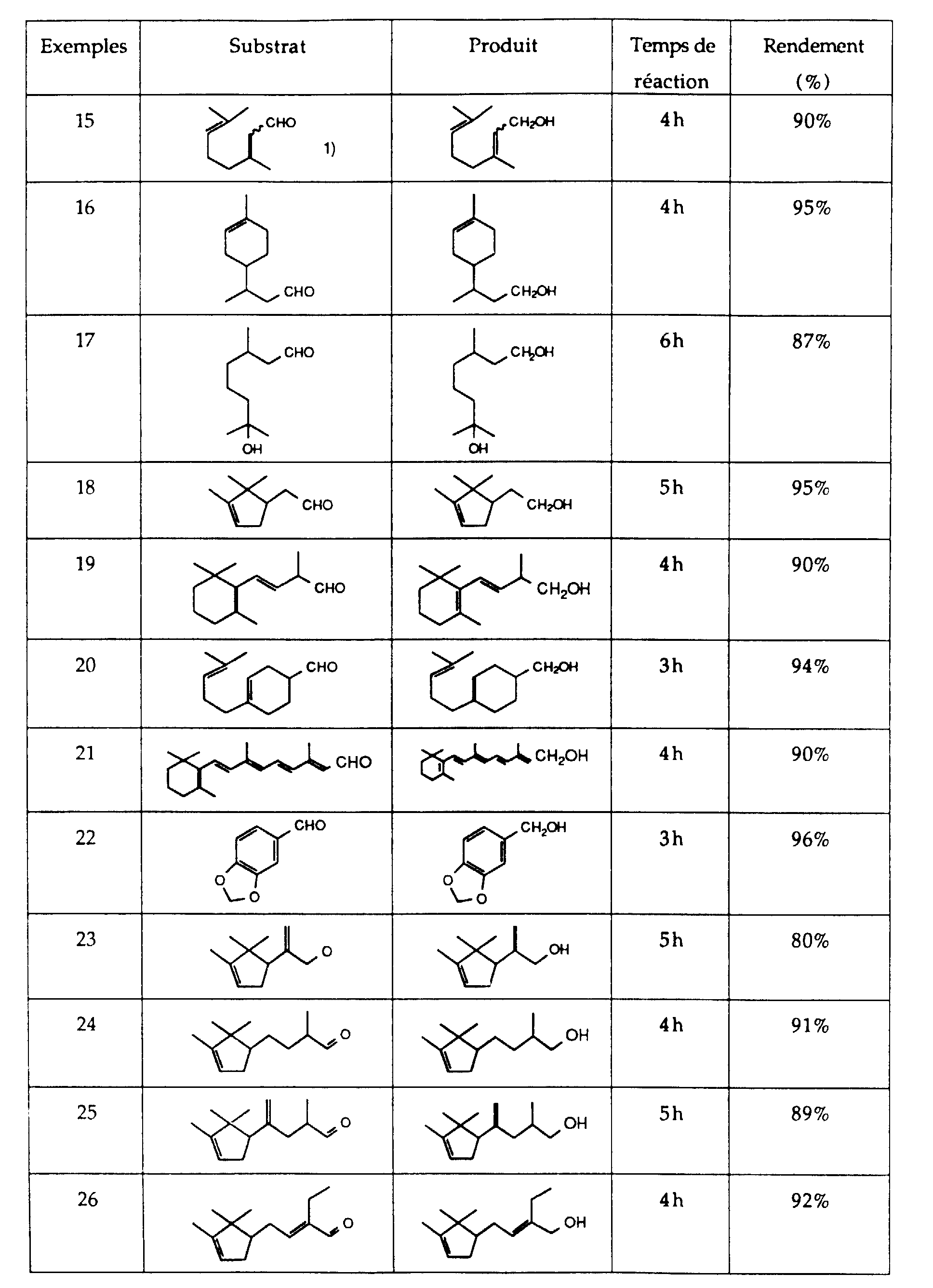

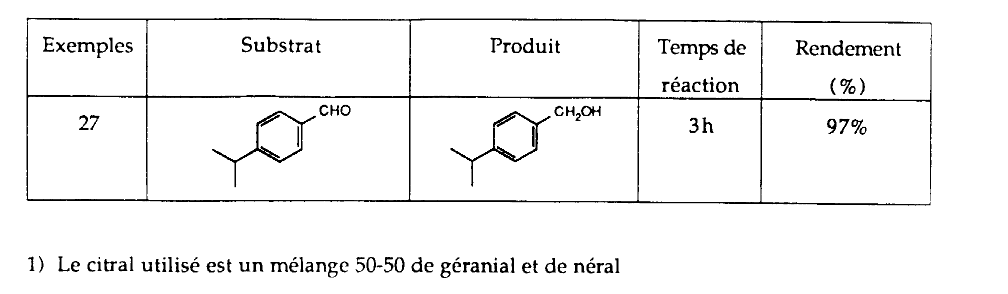

### Réduction des cétones

### Exemple 28

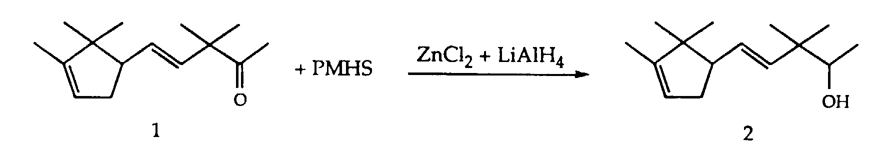

Dans un ballon tricol de 1l, on introduit 50 g d'éther isopropylique, 5 g de LiAlH₄ en solution dans le toluène à 15% (0,01 mole), puis 1,36 g de chlorure de zinc (0,01 mole) et on laisse agiter 15 minutes jusqu'à ce que le dégagement d'hydrogène cesse. Puis on introduit 110 g (0,5 mole) de 3,3-diméthyl-5-(2,2,3-triméthyl-cyclopent-3-én-1-yl)-pent-4-én-2-one (formule 1 ci-dessus), et on porte le milieu réactionnel à reflux. On introduit alors 36 g de PMHS (0,55 mole) en 1 h. On laisse agiter 2 h supplémentaires jusqu'à ce que le contrôle GC indique que tout le substrat a disparu. On refroidit alors le mélange à 20°, on ajoute alors lentement 85 g de potasse aqueuse à 45%, en veillant à ce que la température ne dépasse pas 40°, et on laisse agiter 1 h. On décante le mélange, on sépare la phase aqueuse contenant le silicate de sodium, puis on lave la phase organique avec 100 ml d'eau. On évapore le solvant et on obtient 116 g de produit dont la distillation sur résidus donne 105 g de 3,3-diméthyl-5-(2,2,3-triméthyl-cyclopent-3-én-1-yl)-pent-4-én-2-ol (formule 2 ci-dessus) de pureté supérieure à 98% (rendement = 95% molaire).

### Exemples 29 à 37

Dans un ballon tricol de 250 ml, on charge 50 ml d'éther isopropylique, puis 0,4 g de borohydrure de sodium solide (10 mmole), et 10 mmole de sel ou complexe métallique dont la nature est indiquée dans le tableau. On laisse réagir 30 minutes à reflux jusqu'à ce que le dégagement d'hydrogène cesse, puis on introduit 50 g de cyclohex-1-én-3-one (0,57 mole). On introduit alors dans le mélange réactionnel 37,2 g de PMHS (0,57 mole) en 30 minutes et on contrôle l'avancement de la réaction par GC. On procède alors à l'hydrolyse du milieu réactionnel par 100 g de soude caustique aqueuse à 30% poids, et on récupère le ou les produits formés par distillation comme dans les exemples précédents.

| Exemples | sel métallique 2% molaire | Réducteur 2% molaire | Durée (heures) | Conversion (%) | Cyclohex-1-én-3-ol Sélectivité (%) | Cyclohexanone (%) Sélectivité (%) |
|---|---|---|---|---|---|---|
| 29 | Mn(2-éthylhexanoate)₂ | NaBH₄ | 5 | 95 | 100 | 0 |
| 30 | Co(2-éthylhexanoate)₂ | NaBH₄ | 6 | 97 | 98 | 0 |
| 31 | Fe(2-éthylhexanoate)₂ | NaBH₄ | 8 | 99 | 98 | 0 |
| 32 | CdCl₂ | NaBH₄ | 5 | 24 | 98 | 2 |
| 33 | Cu(2-éthylhexanoate)₂ | NaBH₄ | 20 | 45 | 1 | 99 |
| 34 | Ni(2-éthylhexanoate)₂ | NaBH₄ | 15 | % | 13 | 87 |
| 35 | PdCl₂(PPh₃)₂ | NaBH₄ | 3 | 99 | 21 | 79 |
| 36 | RuCl₂(PPh₃)₃ | NaBH₄ | 2 | 92 | 46 | 54 |
| 37 | Cr(2-éthylhexanoate)₂ | NaBH₄ | 14 | 38 | 74 | 22 |

### Exemples 38 à 47

On procède comme décrit à l'exemple 28 en opérant dans l'éther isopropylique à reflux (68°), mais en utilisant comme catalyseur un mélange de 2% molaire de 2-éthyl-hexanoate de zinc par rapport au substrat et 2% molaire de NaBH₄, On opère avec 0,5 mole de substrat cétonique que l'on réduit par 0,55 mole de PMHS. L'hydrolyse est alors effectuée lorsque le substrat a disparu avec 0,7 mole de potasse aqueuse à 45%. Après décantation et évaporation du solvant, on procède à la distillation de l'alcool formé. Les résultats des essais indiqués dans le tableau montrent que dans tous les cas la réduction s'effectue très sélectivement et avec d'excellents rendements, sans que la stéréochimie de la molécule de départ ne soit affectée.

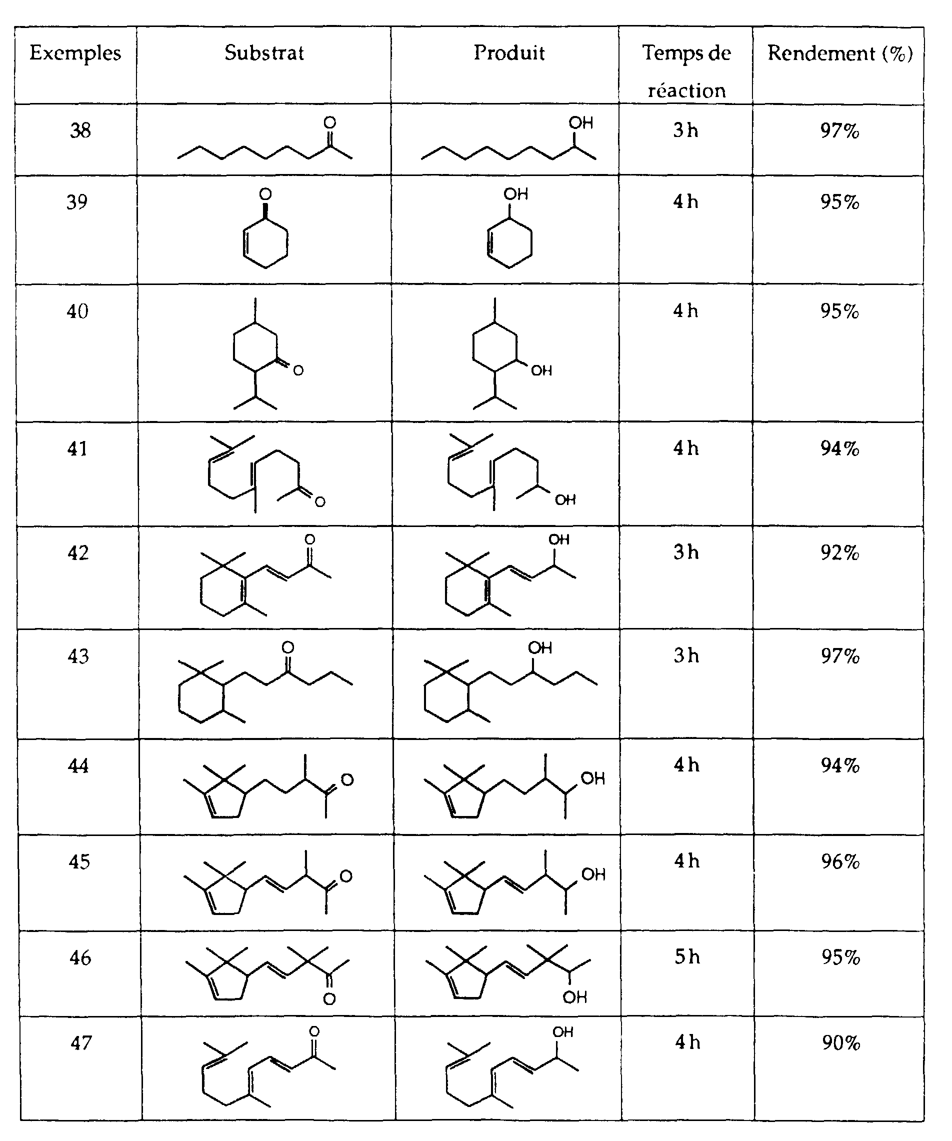

### Réduction des esters et lactones

### Exemple 48

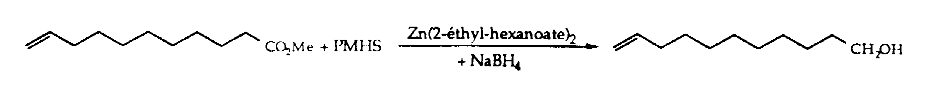

Dans un ballon tricol de 1 l, on introduit 50 g d'éther isopropylique, 0,7 g de NaBH₄ (0,018 mole), puis 1,26 g de 2-éthyl-hexanoate de zinc (0,018 mole) et on laisse agiter 15 minutes jusqu'à ce que le dégagement d'hydrogène cesse. Puis on introduit 120 g (0,6 mole) de 10-undécylénate de méthyle, et on porte le milieu réactionnel à reflux. On introduit alors 90 g de PMHS (1,38 mole) en 1 h. On laisse agiter 2 h supplémentaires sous reflux à 68° jusqu'à ce que le contrôle GC indique que tout le substrat a disparu. On refroidit alors le mélange à 20°, on ajoute alors lentement 300 g de potasse méthanolique à 30% sous bonne agitation, et on laisse agiter 1 h.
On ajoute alors 600 ml d'eau, et on décante le mélange. On sépare la phase aqueuse contenant le silicate de sodium, puis on lave la phase organique avec 100 ml d'eau. On évapore le solvant et on obtient 104 g de produit dont la distillation sur résidus donne 96 g de 10-undécénol de pureté supérieure à 98% (rendement = 94% molaire).

### Exemples 49 à 61

On procède comme décrit à l'exemple 48 en opérant dans l'éther isopropylique à reflux (68°), et en utilisant comme catalyseur un mélange de 2% molaire de 2-éthyl-hexanoate de zinc par rapport au substrat et 2% molaire de NaBH₄, On opère avec 0,5 mole d'ester ou de lactone que l'on réduit par 1,2 mole de PMHS. L'hydrolyse est alors effectuée lorsque le substrat a disparu avec 1,7 mole de potasse alcoolique à 45%. Après décantation et évaporation du solvant, on procède à la distillation de l'alcool formé. Les résultats des essais figurant dans le tableau indiquent que dans tous les cas la réduction des esters et lactones s'effectue très sélectivement et avec d'excellents rendements, sans que la stéréochimie de la molécule de départ ne soit affectée.

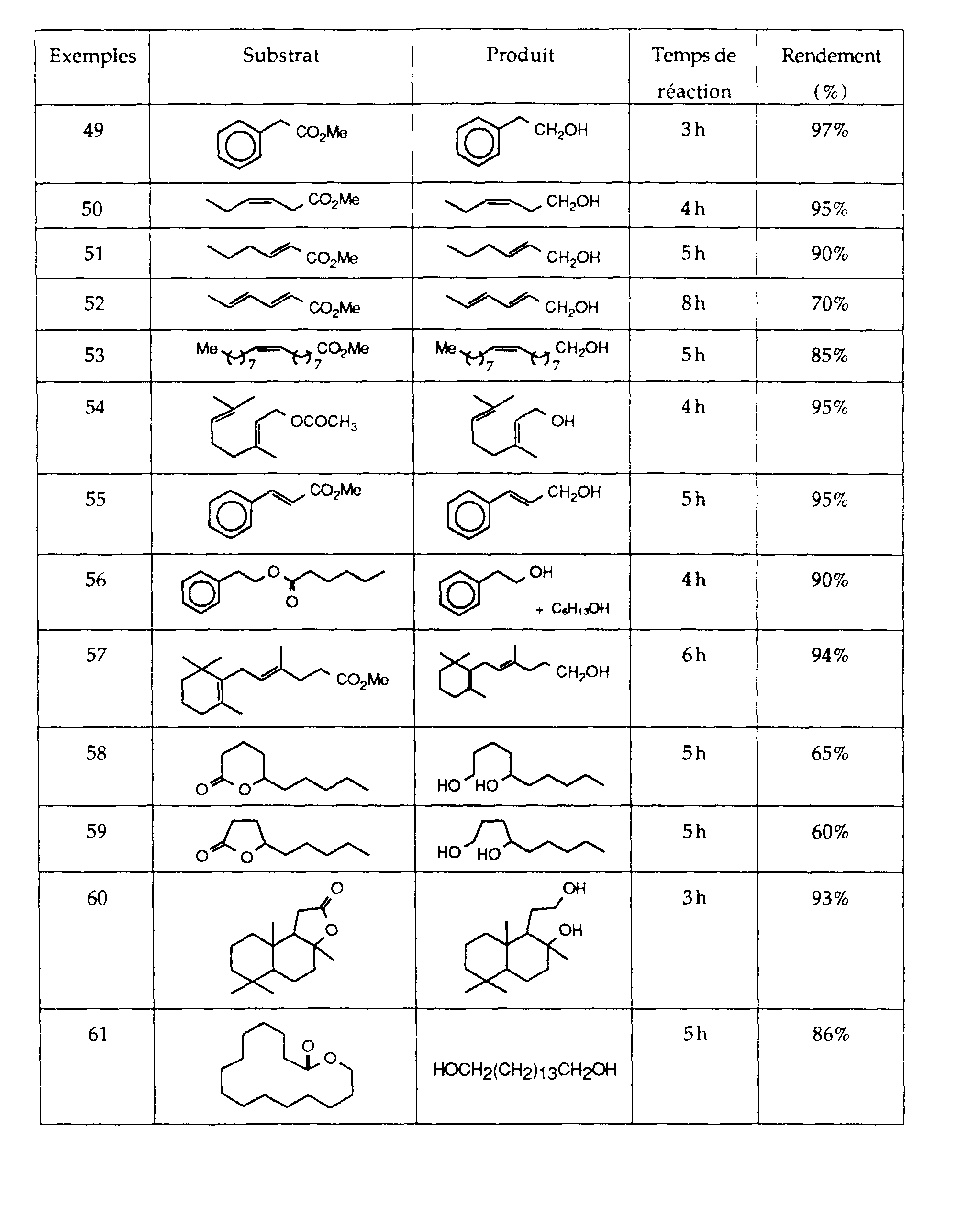

### Réduction des triglycérides

### Exemple 62

Dans un ballon tricol de 1 l, on introduit 400 g de toluène, puis 1,2 g de borohydrure de sodium et 11 g de 2-éthylhexanoate de zinc. On chauffe le mélange à 80°C pendant 30 min environ, jusqu'à ce que le dégagement d'hydrogène cesse. On introduit alors 200 g de trioléine (trioléate de glycerol), puis, en 2 heures, 130 g de PMHS en maintenant la solution à 110°C, à la température de reflux du toluène. On laisse agir 4 heures supplémentaires, jusqu'à ce que l'analyse GC des échantillons hydrolysés par de la potasse méthanolique à 30% indique que la quantité d'alcool oléique formé n'augmente plus.
On verse alors le mélange réactionnel sur 450 g d'une solution de potasse méthanolique à 30% en maintenant la température inférieure à 40°C, et on laisse agir une heure supplémentaire. On ajoute alors à la solution 400 ml d'eau et on laisse décanter. On sépare alors la phase organique puis on évapore le toluène. On distille alors sous vide poussé de 1,33 x 10² Pa (1 mm Hg) à 200-250°C 185 g d'un produit constitué d'alcool oléique (Z-9-octadécén-1-ol) à 96% de pureté.

### Exemple 63

On procède comme dans l'Exemple 62, mais en utilisant 200 g d'huile d'arachide. On obtient par distillation 120 g d'un mélange constitué de 14% de 1-hexadécanol, 55% d'alcool oléique et 17% d'alcool linoléique (Z,Z-9,12-octadécadién-1-ol).

### Exemple 64

On procède comme dans l'Exemple 62, mais en partant de 200 g d'huile de tournesol. On obtient par distillation 110 g d'un mélange constitué de 12% de 1-hexadécanol, 35% d'alcool oléique et 40% d'alcool linoléique (Z,Z-9,12-octadécadién-1-ol).

### Exemple 65

On procède comme dans l'Exemple 62, mais en partant de 200 g d'huile de lin. On obtient par distillation 110 g d'un mélange constitué de 5% de 1-hexadécanol, 18% d'alcool oléique, 17% d'alcool linoléique (Z,Z-9,12-octadécadién-1-ol) et 52% d'alcool linolénique (Z,Z,Z-9,12,15-octadécatrién-1-ol).

### Exemple 66

On procède comme dans l'Exemple 62, mais en partant de 200 g d'huile de colza. On obtient par distillation 110 g d'un mélange constitué de 58% d'alcool oléique, 20% d'alcool linoléique (Z,Z-9,12-octadécadién-1-ol) et 8% d'alcool linolénique (Z,Z,Z-9,12,15-octadécatrién-1-ol).

## Revendications

1. Système réducteur comprenant
a. polyméthylhydroxysilane (PMHS) ;
b. un sel ou complexe métallique de formule MXₙ, dans laquelle M représente un métal de transition sélectionné parmi le groupe constitué par le zinc, le cadmium, le manganèse, le cobalt, le fer, le cuivre, le nickel, le ruthénium et le palladium, X est un anion tel qu'un halogénure, un carboxylate ou un ligand anionique et n est un nombre de 1 à 4 ;
et
c. un agent réducteur sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens.

2. Système réducteur selon la revendication 1, caractérisé en ce qu'il se présente sous une forme comprenant un mélange des composantes b. et c., ou un catalyseur susceptible d'être obtenu par ce mélange.

3. Système réducteur, susceptible d'être obtenu par la réaction de :
a. polyméthylhydroxysilane (PMHS)
avec
b. un catalyseur susceptible d'être obtenu par mélange d'un :
i) sel ou complexe métallique de formule MXₙ, dans laquelle M représente un métal de transition sélectionné parmi le groupe constitué par le zinc, le cadmium, le manganèse, le cobalt, le fer, le cuivre, le nickel, le ruthénium et le palladium, X est un anion tel qu'un halogénure, un carboxylate ou un ligand anionique et n est un nombre de 1 à 4, et
ii) d'un agent réducteur sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens.

4. Système réducteur susceptible d'être mélangé avec le polyméthylhydroxysilane (PMHS) pour effectuer la réduction d'un substrat carbonylé en l'alcool correspondant, comprenant :
a. un catalyseur susceptible d'être obtenu par la réaction d'un :
i) sel ou complexe métallique de formule MXₙ, dans laquelle M représente un métal de transition sélectionné parmi le groupe constitué par le zinc, le cadmium, le manganèse, le cobalt, le fer, le cuivre, le nickel, le ruthénium et le palladium, X est un anion tel qu'un halogénure, un carboxylate ou un ligand anionique et n est un nombre de 1 à 4, et
ii) d'un agent réducteur sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens ; et
b. le substrat carbonylé à être réduit.

5. Système réducteur selon la revendication 4, caractérisé en ce que ledit substrat carbonylé est sélectionné dans le groupe constitué par la 3-méthyl-cyclopenta-1,5-dione, la 3,3-diméthyl-5-(2,2,3-triméthyl-cyclopent-3-én-1-yl)-pent-4-én-2-one et le trans-hex-2-én-1-al, un aldéhyde à chaîne linéaire ou ramifiée parmi les butanal, pentanal, hexanal, heptanal, octanal, décanal et dodécanal, l'acroléine, la méthacroléine, le crotonaldéhyde, le prénal, le citral, le rétinal, l'aldéhyde campholénique, l'aldéhyde cinnamique, l'aldéhyde hexylcinnamique, le formylpinane, le nopal, le benzaldéhyde, l'aldéhyde cuminique, la vanilline, l'aldéhyde salicylique, l'hexan-2-one, l'octan-2-one, le nonan-4-one, la dodécan-2-one, la méthyl vinyl cétone, le mésityl oxyde, l'acétophénone, la cyclopentanone, la cyclohexanone, la cyclododécanone, la cyclohex-1-én-3-one, l'isophorone, l'oxophorone, la carvone et le camphre.

6. Système réducteur selon la revendication 4, caractérisé en ce que le substrat carbonylé est un ester ou une lactone sélectionné dans le groupe constitué par les acétates, propionates, butyrates, isobutyrates, alkyl ou aryl benzoates, acrylates, alkyl ou aryl crotonoates, les alkyl cinnamates, le méthyl cis-3-hexénoate, le méthyl sorbate, le méthyl salicylate, le méthyl 10-undécylénate, le méthyl oléate, le méthyl linoléate ou tout mélange d'esters d'acides gras d'origine naturelle, la caprolactone, la butyrolactone, la dodécalactone, le dikétène et la sclaréolide.

7. Système réducteur selon la revendication 4, caractérisé en ce que le substrat est un triglycéride d'acides gras de formule 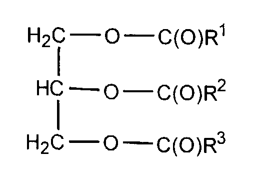 dans laquelle R¹, R² et R³ représentent des restes hydrocarbonés identiques ou différents, saturés ou insaturés, pouvant contenir de 1 à 20 atomes de carbone.

8. Système réducteur selon la revendication 7, caractérisé en ce qu'on utilise une huile végétale en tant que triglycéride d'acide gras.

9. Système réducteur selon la revendication 8, caractérisé en ce que l'huile végétale est choisie parmi la trioléine, l'huile d'arachide, l'huile de tournesol, l'huile de soja, l'huile d'olive, l'huile de colza, l'huile de sésame, l'huile de lin, l'huile de coton, l'huile de coprah, l'huile de pépin de raisins, l'huile de coco et l'huile de palme.

10. Système réducteur selon l'une des revendications 1 à 9, caractérisé en ce que le métal du sel ou complexe métallique est le zinc, le manganèse, le cobalt ou le fer.

11. Système réducteur selon l'une des revendications 1 à 10, caractérisé en ce que l'anion du sel ou complexe métallique est sélectionné parmi le groupe constitué par chlorure, bromure, iodure, carbonate, isocyanate, cyanate, sulfate, phosphate, acétate, propionate, 2-éthylhexanoate, stéarate et naphténate.

12. Système réducteur selon l'une des revendications 1 à 11, caractérisé en ce que l'agent réducteur est le borohydrure de sodium.

13. Système réducteur selon la revendication 12, caractérisé en ce que le sel ou complexe métallique est le Zn(2-éthylhexanoate)₂, le Mn(2-éthylhexanoate)₂, le Co(2-éthylhexanoate)₂ ou le Fe(2-éthylhexanoate)₂.

14. Système réducteur selon l'une des revendications 2 à 13 comprenant en plus un agent complexant du catalyseur résultant de la réaction du sel ou complexe métallique avec l'agent réducteur.

15. Système réducteur selon la revendication 14, caractérisé en ce que l'agent complexant est sélectionné parmi les éthers alcools ou les amino alcools.

16. Système réducteur selon la revendication 15, caractérisé en ce que l'agent complexant est le méthoxyéthanol, le diméthylaminométhanol, le diméthylaminoéthanol, la diéthanolamine ou la triéthanolamine.

17. Système réducteur selon l'une des revendications 2 à 9, caractérisé en ce que la concentration de catalyseur, exprimée en pourcentage molaire de métal par rapport au substrat, est comprise entre 0,1 et 10%.

18. Système réducteur selon l'une des revendications 2 à 9, caractérisé en ce que le PMHS est utilisé en quantité stoïchiométrique par rapport au substrat carbonylé.

19. Système réducteur selon l'une des revendications 2 à 9, caractérisé en ce que le catalyseur est utilisé dans un rapport molaire d'agent réducteur pour métal de 1 à 2.

20. Système catalytique comprenant :
a. un sel ou complexe métallique de formule MXₙ, dans laquelle M représente un métal de transition sélectionné parmi le groupe constitué par le zinc, le cadmium, le manganèse, le cobalt, le fer, le cuivre, le nickel, le ruthénium et le palladium, X est un anion tel qu'un halogénure, un carboxylate ou un ligand anionique et n est un nombre de 1 à 4 ;
b. un agent réducteur sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens ; et
c. un agent complexant sélectionné parmi les éthers alcools et les aminoalcools.

21. Système catalytique adapté à être mélangé pour effectuer la réduction au polyméthylhydroxysilane (PMHS) d'un substrat carbonylé en l'alcool correspondant, caractérisé en ce qu'il est susceptible de se former par mélange :
a. d'un sel ou complexe métallique de formule MXₙ, dans laquelle M représente un métal de transition sélectionné parmi le groupe constitué par le zinc, le cadmium, le manganèse, le cobalt, le fer, le cuivre, le nickel, le ruthénium et le palladium, X est un anion tel qu'un halogénure, un carboxylate ou un ligand anionique et n est un nombre de 1 à 4 ;
et
b. d'un agent réducteur sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens,
en présence d'un agent complexant sélectionné parmi les éthers alcools et les aminoalcools.

22. Système catalytique selon l'une des revendications 20 ou 21, caractérisé en ce que le métal du sel ou complexe métallique est le zinc, le manganèse, le cobalt ou le fer.

23. Système catalytique selon l'une des revendications 20 à 22, caractérisé en ce que l'anion du sel ou complexe métallique est sélectionné parmi le groupe constitué par chlorure, bromure, iodure, carbonate, isocyanate, cyanate, sulfate, phosphate, acétate, propionate, 2-éthylhexanoate, stéarate et naphténate.

24. Système catalytique selon la revendication 23, caractérisé en ce que le sel ou complexe métallique est le Zn(2-éthylhexanoate)₂, le Mn(2-éthylhexanoate)₂, le Co(2-éthylhexanoate)₂ ou le Fe(2-éthylhexanoate)₂.

25. Système catalytique selon l'une des revendications 20 à 24, caractérisé en ce que l'agent complexant est le méthoxyéthanol, le diméthylaminométhanol, le diméthylaminoéthanol, la diéthanolamine ou la triéthanolamine.

26. Système catalytique selon la revendication 25, caractérisé en ce que le rapport molaire entre l'agent réducteur et le métal est de 1 à 2.

27. Système catalytique selon la revendication 20 ou 21, caractérisé en ce que le sel ou complexe métallique est un sel de zinc, de préférence le Zn(2-éthylhexanoate)₂ et l'agent réducteur est le borohydrure de sodium.

28. Procédé pour la préparation d'alcools par la réduction de la fonction carbonyle dans des substrats appartenant à la classe des aldéhydes, cétones, esters ou lactones, pouvant contenir ou non des fonctions insaturées autres que le carbonyle,
caractérisé en ce qu'il comprend :
a. la réaction du substrat carbonylé avec des quantités stoïchiométriques de PMHS en présence d'un système catalytique préparé à partir d'un sel ou complexe métallique et d'un agent réducteur, ledit sel ou complexe métallique étant de formule générale MXₙ dans laquelle M représente un métal de transition choisi parmi le zinc, le cadmium, le manganèse, le cobalt, le fer, le cuivre, le nickel, le ruthénium et le palladium, X un anion de type halogénure, carboxylate ou un ligand anionique, et n un nombre de 1 à 4 ; et l'agent réducteur étant sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens ;
b. l'hydrolyse du siloxane obtenu au moyen d'un agent basique, et
c. la séparation et purification de l'alcool désiré ainsi formé.

29. Procédé selon la revendication 28, caractérisé en ce que le catalyseur est formé in-situ dans le milieu réactionnel ou ex-situ à partir d'un dérivé métallique par traitement avec un agent réducteur.

30. Procédé selon l'une des revendications 28 ou 29, caractérisé en ce qu'on ajoute au milieu de réaction un agent complexant de l'hydrure métallique formé, sélectionné parmi les éthers alcools et les aminoalcools.

31. Procédé selon l'une des revendications 28 à 30, caractérisé en ce que le sel ou complexe métallique est un sel ou complexe de zinc, de manganèse, de cobalt ou de fer, sélectionné parmi les chlorures, bromures, iodures, carbonates, isocyanates, cyanates, sulfates, phosphates, acétates, propionates, 2-éthylhexanoates, stéarates ou naphténates de ces métaux.

32. Procédé selon la revendication 31, caractérisé en ce que le sel métallique est le Zn(2-éthylhexanoate)₂, le Mn(2-éthylhexanoate)₂, le Co(2-éthylhexanoate)₂ ou le Fe(2-éthylhexanoate)₂.

33. Procédé selon l'une des revendications 28 à 32, caractérisé en ce que l'agent réducteur est le borohydrure de sodium.

34. Procédé selon la revendication 30, caractérisé en ce que l'agent complexant est le méthoxyéthanol, le diméthylaminométhanol, le diméthylaminoéthanol, la diéthanolamine ou la triéthanolamine.

35. Procédé selon l'une des revendications 28 à 34, caractérisé en ce que la concentration de catalyseur, exprimée en pourcent molaire du métal par rapport au substrat, est comprise entre 0,1 et 10%.

36. Procédé selon la revendication 28, caractérisé en ce que l'hydrolyse du siloxane obtenu après réduction est effectuée au moyen de traitement du mélange réactionnel avec de la soude, de la potasse, de la chaux ou du carbonate de sodium.

37. Procédé selon la revendication 28, caractérisé en ce que la réduction s'effectue dans un solvant organique inerte choisi parmi les éthers ou les hydrocarbures aliphatiques ou aromatiques.

38. Procédé selon l'une des revendications 28 à 37, caractérisé en ce que la réduction s'effectue à une température comprise entre 0 et 150°C, et de préférence entre 50° et 110°C.

39. Procédé selon l'une des revendications 28 à 38, caractérisé en ce qu'on réduit l'un quelconque des substrats définis aux revendications 5 à 9.

40. Produit de réaction formé lors de la réduction au polyméthylhydroxysilane (PMHS) d'un substrat carbonylé en l'alcool correspondant et avant la récupération de cet alcool par hydrolyse, susceptible d'être obtenu par la réaction dudit substrat carbonylé avec le PMHS, en présence d'un catalyseur obtenu par la réaction :
i) d'un sel ou complexe métallique de formule MXₙ, dans laquelle M représente un métal de transition sélectionné parmi le groupe constitué par le zinc, le cadmium, le manganèse, le cobalt, le fer, le cuivre, le nickel, le ruthénium et le palladium, X est un anion tel qu'un halogénure, un carboxylate ou un ligand anionique et n est un nombre de 1 à 4, et
ii) d'un agent réducteur sélectionné parmi le groupe constitué par les hydrures de lithium, sodium ou potassium, les hydrures de métaux alcalino-terreux, les hydrures de bore, les alkylboranes, les alcoxyboranes, les hydrures d'aluminium, les composés organomagnésiens et les composés organolithiens.

41. Produit selon la revendication 40, caractérisé en ce qu'on utilise un sel ou complexe de zinc, manganèse, cobalt ou fer, sélectionné parmi les chlorures, bromures, iodures, carbonates, isocyanates, cyanates, sulfates, phosphates, acétates, propanoates, 2-éthylhexanoates, stéarates et naphténates de ces métaux.

42. Produit selon la revendication 41, caractérisé en ce que le sel ou complexe métallique est le Zn(2-éthylhexanoate)₂, Mn(2-éthylhexanoate)₂, Co(2-éthylhexanoate)₂ ou le Fe(2-éthylhexanoate)₂ et l'agent réducteur est le borohydrure de sodium.

43. Produit selon la revendication 40, caractérisé en ce que le PMHS est présent en quantités stoïchiométriques par rapport au substrat.

44. Produit selon l'une des revendications 40 à 43, caractérisé en ce que le substrat est l'un quelconque des substrats définis aux revendications 5 à 9.

## Patentansprüche

1. Reduzierendes System, welches aufweist:
a. Polymethylhydroxysilan (PMHS);
b. ein Metallsalz oder einen Metallkomplex mit der Formel MXₙ, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist;
und
c. ein Reduktionsmittel, das aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist.

2. Reduzierendes System nach Anspruch 1, dadurch gekennzeichnet, daß es in einer Form vorliegt, welche eine Mischung der Komponenten b. und c., oder einen durch diese Mischung herstellbaren Katalysator aufweist.

3. Reduzierendes System, das herstellbar ist durch die Umsetzung von:
a. Polymethylhydroxysilan (PMHS) mit
b. einem Katalysator, der herstellbar ist durch Mischen eines:
i) Metallsalzes oder -komplexes mit der Formel MXₙ, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist, und
ii) eines Reduktionsmittels, das aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist.

4. Reduzierendes System, das mit Polymethylhydroxysilan (PMHS) mischbar ist, um die Reduktion eines Carbonylgruppen enthaltenden Substrats zu dem entsprechenden Alkohol durchzuführen, und das aufweist:
a. einen Katalysator, der herstellbar ist durch Umsetzung eines:
i) Metallsalzes oder -komplexes mit der Formel MXₙ, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist, und
ii) eines Reduktionsmittels, das aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist; und
b. das zu reduzierende, Carbonylgruppen enthaltende Substrat.

5. Reduzierendes System nach Anspruch 4, dadurch gekennzeichnet, daß das Carbonylgruppen enthaltende Substrat aus der aus 3-Methyl-cyclopenta-1,5-dion, 3,3-Dimethyl-5-(2,2,3-trimethyl-cyclopent-3-en-1-yl)-pent-4-en-2-on und trans-Hex-2-en-1-al, einem Aldehyd mit linearer oder verzweigter Kette ausgewählt aus Butanal, Pentanal, Hexanal Heptanal, Octanal, Decanal und Dodecanal, Acrolein, Methacrolein, Crotonaldehyd, Prenal, Citral, Retinal, Campholenaldehyd, Zimtaldehyd, Hexylzimtaldehyd, Formylpinan, Nopal, Benzaldehyd, Cuminaldehyd, Vanillin, Salicylaldehyd, Hexan-2-on, Octan-2-on, Nonan-4-on, Dodecan-2-on, Methylvinylketon, Mesityloxid, Acetophenon, Cyclopentanon, Cyclohexanon, Cyclododecanon, Cyclohex-1-en-3-on, Isophoron, Oxophoron, Carvon und Campher bestehenden Gruppe ausgewählt ist.

6. Reduzierendes System nach Anspruch 4, dadurch gekennzeichnet, daß das Carbonylgruppen enthaltende Substrat ein Ester oder ein Lacton ist, der bzw. das aus der aus Acetaten, Propionaten, Butyraten, Isobutyraten, Alkyl- oder Arylbenzoaten, Acrylaten, Alkyl- oder Arylcrotonaten, Alkylcinnamaten, Methyl-cis-3-hexenoat, Methylsorbat, Methylsalicylat, Methyl-10-undecylenat, Methyloleat, Methyllinoleat oder jeglicher Mischung von Estern von Fettsäuren natürlichen Ursprungs, Caprolacton, Butyrolacton, Dodecalacton, Diketen und Sclareolid bestehenden Gruppe ausgewählt ist.

7. Reduzierendes System nach Anspruch 4, dadurch gekennzeichnet, daß das Substrat ein Fettsäuretriglycerid mit der Formel 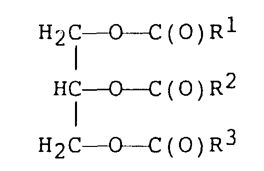 ist, in der R¹, R² und R³ für gleiche oder verschiedene, gesättigte oder ungesättigte Kohlenwasserstoffreste stehen, welche von 1 bis 20 Kohlenstoffatome enthalten können.

8. Reduzierendes System nach Anspruch 7, dadurch gekennzeichnet, daß ein pflanzliches Öl als Fettsäuretriglycerid verwendet wird.

9. Reduzierendes System nach Anspruch 8, dadurch gekennzeichnet, daß das pflanzliche Öl aus Triolein, Erduß-öl, Sonnenblumenöl, Sojaöl, Olivenöl, Rapsöl, Sesamöl, Leinöl, Baumwollöl, Kopraöl, Traubenkernöl, Kokosöl und Palmöl ausgewählt ist.

10. Reduzierendes System nach einem der Ansprüche 1 bis 9, dadurch gekennzeichnet, daß das Metall des Metallsalzes oder -komplexes Zink, Mangan, Cobalt oder Eisen ist.

11. Reduzierendes System nach einem der Ansprüche 1 bis 10, dadurch gekennzeichnet, daß das Anion des Metallsalzes oder -komplexes aus der aus Chlorid, Bromid, Iodid, Carbonat, Isocyanat, Cyanat, Sulfat, Phosphat, Acetat, Propionat, 2-Ethylhexanoat, Stearat und Naphthenat bestehenden Gruppe ausgewählt ist.

12. Reduzierendes System nach einem der Ansprüche 1 bis 11, dadurch gekennzeichnet, daß das Reduktionsmittel Natriumborhydrid ist.

13. Reduzierendes System nach Anspruch 12, dadurch gekennzeichnet, daß das Metallsalz oder der Metallkomplex Zn(2-ethylhexanoat)₂, Mn(2-ethylhexanoat)₂, Co(2-ethylhexanoat)₂ oder Fe(2-ethylhexanoat)₂ ist.

14. Reduzierendes System nach einem der Ansprüche 2 bis 13, welches des weiteren einen Komplexbildner für den aus der Umsetzung des Metallsalzes oder -komplexes mit dem Reduktionsmittel resultierenden Katalysator beinhaltet.

15. Reduzierendes System nach Anspruch 14, dadurch gekennzeichnet, daß der Komplexbildner aus den Etheralkoholen oder Aminoalkoholen ausgewählt ist.

16. Reduzierendes System nach Anspruch 15, dadurch gekennzeichnet, daß der Komplexbildner Methoxyethanol, Dimethylaminomethanol, Dimethylaminoethanol, Diethanolamin oder Triethanolamin ist.

17. Reduzierendes System nach einem der Ansprüche 2 bis 9, dadurch gekennzeichnet, daß die Konzentration des Katalysators, ausgedrückt in Molprozent von Metall in Bezug auf das Substrat, zwischen 0,1 und 10% liegt.

18. Reduzierendes System nach einem der Ansprüche 2 bis 9, dadurch gekennzeichnet, daß das PMHS in stöchiometrischer Menge in Bezug auf das Carbonylgruppen enthaltende Substrat verwendet wird.

19. Reduzierendes System nach einem der Ansprüche 2 bis 9, dadurch gekennzeichnet, daß der Katalysator in einem Molverhältnis von Reduktionsmittel zu Metall von 1 bis 2 verwendet wird.

20. Katalytisches System, welches aufweist:
a. ein Metallsalz oder einen Metallkomplex mit der Formel MXₙ, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist;
b. ein Reduktionsmittel, das aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist; und
c. einen Komplexbildner, der aus den Etheralkoholen und den Aminoalkoholen ausgewählt ist.

21. Katalytisches System, das zum Mischen geeignet ist, um die Reduktion mit Polymethylhydroxysilan (PMHS) eines Carbonylgruppen enthaltenden Substrates zu dem entsprechenden Alkohol durchzuführen, dadurch gekennzeichnet, daß seine Bildung möglich ist durch Mischen:
a. eines Metallsalzes oder -komplexes mit der Formel MXₙ, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist;
und
b. eines Reduktionsmittels, das aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist,
in Gegenwart eines unter den Etheralkoholen und den Aminoalkoholen ausgewählten Komplexbildners.

22. Katalytisches System nach einem der Ansprüche 20 oder 21, dadurch gekennzeichnet, daß das Metall des Metallsalzes oder -komplexes Zink, Mangan, Cobalt oder Eisen ist.

23. Katalytisches System nach einem der Ansprüche 20 bis 22, dadurch gekennzeichnet, daß das Anion des Metallsalzes oder -komplexes aus der aus Chlorid, Bromid, Iodid, Carbonat, Isocyanat, Cyanat, Sulfat, Phosphat, Acetat, Propionat, 2-Ethylhexanoat, Stearat und Naphthenat bestehenden Gruppe ausgewählt ist.

24. Katalytisches System nach Anspruch 23, dadurch gekennzeichnet, daß das Metallsalz oder der Metallkomplex Zn(2-ethylhexanoat)₂, Mn(2-ethylhexanoat)₂, Co(2-ethylhexanoat)₂ oder Fe(2-ethylhexanoat) ist.

25. Katalytisches System nach einem der Ansprüche 20 bis 24, dadurch gekennzeichnet, daß der Komplexbildner Methoxyethanol, Dimethylaminomethanol, Dimethylaminoethanol, Diethanolamin oder Triethanolamin ist.

26. Katalytisches System nach Anspruch 25, dadurch gekennzeichnet, daß das Molverhältnis zwischen dem Reduktionsmittel und dem Metall von 1 bis 2 ist.

27. Katalytisches System nach Anspruch 20 oder 21, dadurch gekennzeichnet, daß das Metallsalz oder der Metallkomplex ein Zinksalz, bevorzugt Zn(2-ethylhexanoat)₂, und das Reduktionsmittel Natriumborhydrid ist.

28. Verfahren zur Herstellung von Alkoholen mittels Reduzierung der Carbonylfunktion in Substraten, welche der Klasse der Aldehyde, Ketone, Ester oder Lactone angehören und gegebenenfalls andere ungesättigte Gruppen als Carbonyl enthalten können,
dadurch gekennzeichnet, daß es aufweist:
a. die Umsetzung des Carbonylgruppen enthaltenden Substrats mit stöchiometrischen Mengen von PMHS in Gegenwart eines ausgehend von einem Metallsalz oder -komplex und einem Reduktionsmittel gebildeten katalytischen Systems, wobei das Metallsalz oder der Metallkomplex die allgemeine Formel MXₙ besitzt, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist; und das Reduktionsmittel aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist;
b. die Hydrolyse des erhaltenen Siloxans, mit Hilfe eines basischen Mittels, und
c. die Abtrennung und Reinigung des hierdurch gebildeten angestrebten Alkohols.

29. Verfahren nach Anspruch 28, dadurch gekennzeichnet, daß der Katalysator in situ im Reaktionsmedium oder ex situ ausgehend von einem Metallderivat durch Behandlung mit einem Reduktionsmittel gebildet wird.

30. Verfahren nach einem der Ansprüche 28 oder 29, dadurch gekennzeichnet, daß dem Reaktionsmedium ein unter den Etheralkoholen und den Aminoalkoholen ausgewählter Komplexbildner für das gebildete Metallhydrid zugegeben wird.

31. Verfahren nach einem der Ansprüche 28 bis 30, dadurch gekennzeichnet, daß das Metallsalz oder der Metallkomplex ein Salz oder Komplex von Zink, Mangan, Cobalt oder Eisen ist, das bzw. der aus den Chloriden, Bromiden, Iodiden, Carbonaten, Isocyanaten, Cyanaten, Sulfaten, Phosphaten, Acetaten, Propionaten, 2-Ethylhexanoaten, Stearaten oder Naphthenaten dieser Metalle ausgewählt ist.

32. Verfahren nach Anspruch 31, dadurch gekennzeichnet, daß das Metallsalz Zn(2-ethylhexanoat)₂, Mn(2-ethylhexanoat)₂, Co(2-ethylhexanoat)₂ oder Fe(2-ethylhexanoat)₂ ist.

33. Verfahren nach einem der Ansprüche 28 bis 32, dadurch gekennzeichnet, daß das Reduktionsmittel Natriumborhydrid ist.

34. Verfahren nach Anspruch 30, dadurch gekennzeichnet, daß der Komplexbildner Methoxyethanol, Dimethylaminomethanol, Dimethylaminoethanol, Diethanolamin oder Triethanolamin ist.

35. Verfahren nach einem der Ansprüche 28 bis 34, dadurch gekennzeichnet, daß die Konzentration des Katalysators, ausgedrückt in Molprozent des Metalls in Bezug auf das Substrat, zwischen 0,1 und 10% liegt.

36. Verfahren nach Anspruch 28, dadurch gekennzeichnet, daß die Hydrolyse des nach der Reduktion erhaltenen Siloxans mittels Behandlung der Reaktionsmischung mit Ätznatron, Ätzkali, Kalk oder Natriumcarbonat durchgeführt wird.

37. Verfahren nach Anspruch 28, dadurch gekennzeichnet, daß die Reduktion in einem unter den Ethern oder den aliphatischen oder aromatischen Kohlenwasserstoffen ausgewählten, inerten organischen Lösungsmittel stattfindet.

38. Verfahren nach einem der Ansprüche 28 bis 37, dadurch gekennzeichnet, daß die Reduktion bei einer Temperatur stattfindet, die zwischen 0 und 150°C und bevorzugt zwischen 50° und 110°C liegt.

39. Verfahren nach einem der Ansprüche 28 bis 38, dadurch gekennzeichnet, daß eines der in den Ansprüchen 5 bis 9 definierten Substrate reduziert wird.

40. Reaktionsprodukt, das bei der Reduktion mit Polymethylhydroxysilan (PMHS) eines Carbonylgruppen enthaltenden Substrats zu dem entsprechenden Alkohol und vor der Gewinnung dieses Alkohols mittels Hydrolyse gebildet wird, das mittels Umsetzung des Carbonylgruppen enthaltenden Substrats mit dem PMHS in Gegenwart eines Katalysators herstellbar ist, der erhalten wird durch die Umsetzung:
i) eines Metallsalzes oder -komplexes mit der Formel MXₙ, in der M für ein Übergangsmetall steht, welches aus der aus Zink, Cadmium, Mangan, Cobalt, Eisen, Kupfer, Nickel, Ruthenium und Palladium bestehenden Gruppe ausgewählt ist, X ein Anion wie ein Halogenid, ein Carboxylat oder ein anionischer Ligand ist, und n eine Zahl von 1 bis 4 ist, und
ii) eines Reduktionsmittels, das aus der aus den Hydriden von Lithium, Natrium oder Kalium, den Hydriden von Erdalkalimetallen, den Borhydriden, Alkylboranen, Alkoxyboranen, Aluminiumhydriden, Organomagnesiumverbindungen und Organolithiumverbindungen bestehenden Gruppe ausgewählt ist.

41. Produkt nach Anspruch 40, dadurch gekennzeichnet, daß ein Salz oder Komplex von Zink, Mangan, Cobalt oder Eisen verwendet wird, das bzw. der aus den Chloriden, Bromiden, Iodiden, Carbonaten, Isocyanaten, Cyanaten, Sulfaten, Phosphaten, Acetaten, Propionaten, 2-Ethylhexanoaten, Stearaten oder Naphthenaten dieser Metalle ausgewählt ist.

42. Produkt nach Anspruch 41, dadurch gekennzeichnet, daß das Metallsalz oder der Metallkomplex Zn(2-ethylhexanoat)₂, Mn(2-ethylhexanoat)₂, Co(2-ethylhexanoat)₂ oder Fe(2-ethylhexanoat)₂ ist, und das Reduktionsmittel Natriumborhydrid ist.

43. Produkt nach Anspruch 40, dadurch gekennzeichnet, daß das PMHS in stöchiometrischen Mengen in Bezug auf das Substrat vorliegt.

44. Produkt nach einem der Ansprüche 40 bis 43, dadurch gekennzeichnet, daß das Substrat eines der in den Ansprüchen 5 bis 9 definierten Substrate ist.

## Claims

1. Reductive system, comprising :
a. polymethylhydroxysilane (PMHS) ;
b. a metallic salt or complex of formula MXₙ, wherein M represents a transition metal selected from the group consisting of zinc, cadmium, manganese, cobalt, iron, copper, nickel, ruthenium and palladium, X is an anion such as a halide, a carboxylate or an anionic ligand, and n is a number from 1 to 4 ; and
c. a reducing agent selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds.

2. The reductive system according to claim 1, characterized in that it is in a form comprising a mixture of the components a. and c., or a catalyst susceptible of being obtained by their admixture.

3. Reductive system susceptible of being obtained by the reaction of
a. polymethylhydroxysilane (PMHS) ;
with
b. a catalyst susceptible of being obtained by admixture of:
i) a metallic salt or complex of formula MXₙ, wherein M represents a transition metal selected from the group consisting of zinc, cadmium, manganese, cobalt, iron, copper, nickel, ruthenium and palladium, X is an anion such as a halide, a carboxylate or an anionic ligand, and n is a number from 1 to 4 ; and
ii) a reducing agent selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds.

4. Reductive system capable of being mixed together with polymethylhydroxysilane (PMHS) to effect the reduction of a substrate containing a CO-group into the corresponding alcohol, comprising :
a. a catalyst susceptible of being obtained by the reaction of :
i) a metallic salt or complex of formula MXₙ, wherein M represents a transition metal selected from the group consisting of zinc, cadmium, manganese, cobalt, iron, copper, nickel, ruthenium and palladium, X is an anion such as a halide, a carboxylate or an anionic ligand, and n is a number from 1 to 4 ;
ii) a reducing agent selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds ;
and
b. the substrate containing a CO-group to be reduced.

5. Reductive system according to claim 4, characterized in that said substrate containing a CO-group is selected from the group consisting of 3-methylcyclopenta-1,5-dione, 3,3-dimethyl-5-(2,2,3-trimethyl-cyclopent-3-en-1-yl)-pent-4-en-2-one and trans-hex-2-en-1-al, a linear or branched aldehyde selected from butanal, pentanal, hexanal, heptanal, octanal, decanal and dodecanal, acroleine, methacroleine, crotonaldehyde, prenal, citral, retinal, campholenic aldehyde, cinnamic aldehyde, hexylcinnamic aldehyde, formylpinane, nopal, benzaldehyde, cuminic aldehyde, vanilline, salicylic aldehyde, hexan-2-one, octan-2-one, nonan-4-one, dodecan-2-one, methyl vinyl ketone, mesityl oxide, acetophenone, cyclopentanone, cyclohexanone, cyclododecanone, cyclohex-1-en-3-one, isophorone, oxophorone, carvone and camphor.

6. Reductive system according to claim 4, characterized in that said substrate containing a CO-group is an ester or a lactone selected from the group consisting of the acetates, propanoates, butyrates, isobutyrates, alkyl and aryl benzoates, acrylates, alkyl and aryl crotonoates, alkyl cinnamates, methyl cis-3-hexenoate, methyl sorbate, methyl salicylate, methyl 10-undecylenate, methyl oleate, methyl linoleate, any mixture of fatty acid esters of natural origin, caprolactone, butyrolactone, dodecalactone, diketene and sclareolide.

7. Reductive system according to claim 4, characterized in that the substrate is a fatty acid triglyceride of formula 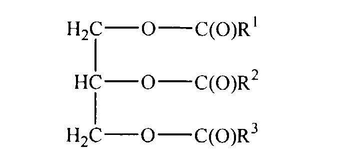 wherein R¹, R² and R³ represent identical or distinct hydrocarbon rests, saturated or unsaturated, comprising from 1 to 20 carbon atoms.

8. Reductive system according to claim 7, characterized in that a vegetable oil is used as the triglyceride of a fatty acid.

9. Reductive system according to claim 8, characterized in that the vegetable oil is selected amongst trioleine, peanut oil, sunflower oil, soya oil, olive oil, colza oil, sesame oil, linseed oil, cotton oil, copra oil, grape seeds oil, coconut oil and palm oil.

10. Reductive system according to any one of claims 1 to 9, characterized in that the transition metal in the metallic salt or complex is zinc, manganese, cobalt or iron.

11. Reductive system according to any one of claims 1 to 10, characterized in that the anion in the metallic salt or complex is selected from the group consisting of chloride, bromide, iodide, carbonate, isocyanate, cyanate, sulfate, phosphate, acetate, propanoate, 2-ethylhexanoate, stearate and naphthenate.

12. Reductive system according to any one of claims 1 to 11, characterized in that the reducing agent is sodium borohydride.

13. Reductive system according to claim 12, characterized in that the metallic salt or complex is Zn(2-ethylhexanoate)₂, Mn(2-ethylhexanoate)₂, Co(2-ethylhexanoate)₂ or Fe(2-ethylhexanoate)₂.

14. Reductive system according to any one of claims 2 to 13, further comprising a complexing agent of the catalyst resulting from the reaction of the metallic salt or complex with the reducing agent.

15. Reductive system according to claim 14, characterized in that the complexing agent is selected from the group consisting of ether alcohols and amino alcohols.

16. Reductive system according to claim 15, characterized in that the complexing agent is methoxyethanol, dimethylaminomethanol, dimethylaminoethanol, diethanolamine or triethanolamine.

17. Reductive system according to any one of claims 2 to 9, characterized in that the concentration of catalyst, expressed in molar percent of metal relative to substrate, is between 0.1 and 10%.

18. Reductive system according to any one of claims 2 to 9, characterized in that PMHS is used in stoichiometric amounts relative to said substrate containing a CO-group.

19. Reductive system according to any one of claims 2 to 9, characterized in that the catalyst is used in a molar ratio of reducing agent relative to metal of 1 to 2.

20. Catalytic system comprising
a. a metallic salt or complex of formula MXₙ, wherein M represents a transition metal selected from the group consisting of zinc, cadmium, manganese, cobalt, iron, copper, nickel, ruthenium and palladium, X is an anion such as a halide, a carboxylate or an anionic ligand, and n is a number from 1 to 4 ; and
b. a reducing agent selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds ;
c. a complexing agent selected amongst the ether alcohols and the amino alcohols.

21. Catalytic system adapted to be admixed to effect the reduction of a substrate containing a CO-group to the corresponding alcohol by polymethylhydroxysilane (PMHS), characterized in that it is susceptible of being formed by admixture of :
a. a metallic salt or complex of formula MXₙ, wherein M represents a transition metal selected from the group consisting of zinc, cadmium, manganese, cobalt, iron, copper, nickel, ruthenium and palladium, X is an anion such as a halide, a carboxylate or an anionic ligand, and n is a number from 1 to 4 ;
and
b. a reducing agent selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds ;
in the presence of a complexing agent selected amongst the ether alcohols and the amino alcohols.

22. Catalytic system according to claim 20 or 21, characterized in that the transition metal in the metallic salt or complex is zinc, manganese, cobalt or iron.

23. Catalytic system according to any one of claims 20 to 22, characterized in that the anion in the metallic salt or complex is selected from the group consisting of chloride, bromide, iodide, carbonate, isocyanate, cyanate, sulfate, phosphate, acetate, propanoate, 2-ethyhexanoate, stearate and naphthenate.

24. Catalytic system according to claim 23, characterized in that the metallic salt or complex is Zn(2-ethylhexanoate)₂, Mn(2-ethylhexanoate)₂, Co(2-ethylhexanoate)₂ or Fe(2-ethylhexanoate)₂.

25. Catalytic system according to any one of claims 20 to 24, characterized in that the complexing agent is methoxyethanol, dimethylaminomethanol, dimethylaminoethanol, diethanolamine or triethanolamine.

26. Catalytic system according to claim 25, characterized in that the molar ratio between the reducing agent and the metal is from 1 to 2.

27. Catalytic system according to claim 20 or 21, characterized in that the metallic salt or complex is a zinc salt, preferably Zn(2-ethylhexanoate)₂, and the reducing agent is sodium borohydride.

28. Process for the preparation of alcohols by reduction of the carbonyl function in substrates belonging to the class of aldehydes, ketones, esters or lactones optionally containing unsaturated functions other than the carbonyl group, characterized in that it comprises :
a. the reaction of the substrate containing a CO-group with stoichiometric amounts of PMHS in the presence of a catalytic system prepared from a metallic salt or complex and a reducing agent, said metallic salt or complex having the general formula MXₙ, wherein M represents a transition metal selected from the group consisting of zinc, cadmium, manganese, cobalt, iron, copper, nickel, ruthenium and palladium, X an anion such as a halide, a carboxylate or an anionic ligand, and n a number comprised between 1 and 4 ; and the reducing agent being selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds ;
b. the hydrolysis of the obtained siloxane by means of a basic agent, and
c. the separation and purification of the desired alcohol thus formed.

29. Process according to claim 28, characterized in that the catalyst is formed in-situ in the reaction medium or ex-situ from a metallic derivative by treatment with a reducing agent.

30. Process according to claim 28 or 29, characterized in that there is added to the reaction medium a complexing agent of the formed metallic hydride, selected from the ether alcohols and the amino alcohols.

31. Process according to any one of claims 28 to 30, characterized in that the metallic salt or complex is a salt or complex of zinc, manganese, cobalt or iron, selected from the group consisting of the chlorides, bromides, iodides, carbonates, isocyanates, cyanates, sulfates, phosphates, acetates, propanoates, 2-ethylhexanoates, stearates and naphthenates of said metals.

32. Process according to claim 30, characterized in that the metallic salt or complex is Zn(2-ethylhexanoate)₂, Mn(2-ethylhexanoate)₂, Co(2-ethylhexanoate)₂ or Fe(2-ethylhexanoate)₂.

33. Process according to any one of claims 28 to 32, characterized in that the reducing agent is sodium borohydride.

34. Process according to claim 30, characterized in that the complexing agent is methoxyethanol, dimethylaminomethanol, dimethylaminoethanol, diethanolamine or triethanolamine.

35. Process according to any one of claims 28 to 34, characterized in that the concentration of the catalyst, expressed in molar percent of metal relative to the substrate, is comprised between 0.1 and 10%.

36. Process according to claim 28, characterized in that the hydrolysis of the siloxane obtained after reduction is carried out by treatment of the reaction mixture with caustic soda, potash, lime or sodium carbonate.

37. Process according to claim 28, characterized in that the reduction is carried ou in an inert organic solvent selected amongst the ethers or the aliphatic or aromatic hydrocarbons.

38. Process according to any one of claims 28 to 37, characterized in that the reduction is carried out at a temperature comprised between 0° and 150°C, and preferably between 50° and 110°C.

39. Process according to any one of claims 28 to 38, characterized in that there is reduced any one of the substrates defined in claims 5 to 9.

40. Reaction product formed upon the reduction by means of polymethylhydroxysilane (PMHS) of a substrate containing a CO-group into the corresponding alcohol and before the recovery of said alcohol via hydrolysis, said reaction product being obtainable by the reaction of said substrate containing a CO-group with PMHS in the presence of a catalyst obtained by the reaction of:
a. a metallic salt or complex of formula MXₙ, wherein M represents a transition metal selected from the group consisting of zinc, cadmium, manganese, cobalt, iron, copper, nickel, ruthenium and palladium, X is an anion such as a halide, a carboxylate or an anionic ligand, and n is a number from 1 to 4 ; and
b. a reducing agent selected from the group consisting of the lithium, sodium and potassium hydrides, the alkaline earth metal hydrides, the boron hydrides, the alkylboranes, the alkoxyboranes, the aluminum hydrides, the organomagnesium compounds and the organolithium compounds.

41. Product according to claim 40, characterized in that there is used a salt or complex of zinc, manganese, cobalt or iron, selected amongst the chlorides, bromides, iodides, carbonates, isocyanates, cyanates, sulfates, phosphates, acetates, propanoates, 2-ethylhexanoates, stearates and naphthenates of these metals.

42. Product according to claim 41, characterized in that the metallic salt or complex is Zn(2-ethylhexanoate)₂, Mn(2-ethylhexanoate)₂, Fe(2-ethylhexanoate)₂ or Co(2-ethylhexanoate)₂ and the reducing agent is sodium borohydride.

43. Product according to claim 40, characterized in that the PMHS is present in stoichiometric amounts relative to the substrate.

44. Product according to any one of claims 40 to 43, characterized in that the substrate is any one of the substrates defined in claims 5 to 9.
